(19) Europäisches Patentamt
European Patent Office
Office européen des brevets

(11) Veröffentlichungsnummer: **0 195 971 B1**

# (12) EUROPÄISCHE PATENTSCHRIFT

(45) Veröffentlichungstag der Patentschrift: **18.10.89**

(51) Int. Cl.⁴: **C 07 C 129/08**, C 07 C 127/26, C 07 C 149/437, C 07 C 155/02

(21) Anmeldenummer: **86103076.5**

(22) Anmeldetag: **07.03.86**

## (54) Verfahren zur Herstellung von substituierten Guanidinen.

(30) Priorität: **23.03.85 DE 3510684**

(43) Veröffentlichungstag der Anmeldung: **01.10.86 Patentblatt 86/40**

(45) Bekanntmachung des Hinweises auf die Patenterteilung: **18.10.89 Patentblatt 89/42**

(84) Benannte Vertragsstaaten: **CH DE FR GB IT LI**

(56) Entgegenhaltungen:
**THE JOURNAL OF THE AMERICAN CHEMICAL SOCIETY, Band 48, Nr. 7, Juli 1926, Seiten 2176-2179, Washington, D.C., US; S. BASTERFIELD et al.: "Studies in urethans I. Mono- and dicarbethoxy-guanidines: dicarbethoxy-ethyl-iso-urea""**

(73) Patentinhaber: **BAYER AG, D-5090 Leverkusen 1 Bayerwerk (DE)**

(72) Erfinder: **Diehr, Hans-Joachim, Dr., Höhe 35, D-5600 Wuppertal 11 (DE)**

EP 0 195 971 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von substituierten Guanidinen. Zwischenprodukte welche für die Durchführung des Verfahrens verwendet werden können, sowie Verfahren zur Herstellung der Zwischenprodukte.

Es ist bereits bekannt, dass man z.B. substituierte Phenylguanidine erhalten kann, wenn man substituierte Anilinderivate mit Isothioharnstoffen umsetzt. Nachteilig bei diesem Verfahren ist die starke Umweltbelastung, weil als Nebenprodukte Alkylmercaptane entstehen (vergl. z.B. EP-OS 27 646). Weiterhin ist die Aufarbeitung der Endprodukte durch die Verunreinigung mit Alkylmercaptanen sehr aufwendig und verursacht eine Reduzierung der Ausbeute (vergl. z.B. DE-AS 2 423 679).

Die Erfindung betrifft:

1. Ein neues Verfahren zur Herstellung von substituierten Guanidinen der Formel (I).

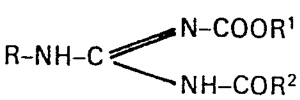

(I)

in welcher

R für einen Rest 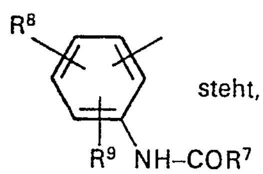 steht, wobei

$R^7$ für Wasserstoff oder für gegebenenfalls substituierte Reste aus der Reihe $C_{1-6}$-Alkyl, $C_{2-7}$-Alkenyl, $C_2$–$C_6$-Alkinyl, $C_1$–$C_6$-Alkoxy, $C_3$–$C_8$-Cycloalkyl, Phenyl und Phenoxy steht,

$R^8$ für Wasserstoff, Halogen, $C_1$–$C_4$-Alkyl oder $C_1$–$C_4$-Alkoxy steht und

$R^9$ für Wasserstoff oder für gegebenenfalls durch Halogen, Cyano, Nitro, Amino, $C_1$–$C_4$-Alkyl, $C_1$–$C_4$-Alkoxy, $C_1$–$C_4$-Alkylthio, Halogen-$C_1$–$C_4$-Alkyl, Halogen-$C_1$–$C_4$-Alkoxy, Halogen-$C_1$–$C_4$-Alkylthio und/oder $C_1$–$C_4$-Alkylcarbonylamino substituierte Reste aus der Reihe Phenoxy, Phenylthio, Benzoyl, Phenylsulfinyl und Phenylsulfonyl; sowie für gegebenenfalls durch Halogen, Cyano, $C_1$–$C_4$-Alkoxy und/oder $C_1$–$C_4$-Alkylthio substituierte Reste aus der Reihe $C_1$–$C_6$-Alkyl, $C_1$–$C_6$-Alkoxy, $C_1$–$C_6$-Alkylthio, $C_1$–$C_6$-Alkylsulfinyl, $C_1$–$C_6$-Alkylsulfonyl, $C_2$–$C_6$-Alkenyloxy, $C_2$–$C_6$-Alkenylthio, $C_2$–$C_6$-Alkenylsulfinyl, $C_2$–$C_6$-Alkenylsulfonyl, $C_2$–$C_6$-Alkinyloxy, $C_2$–$C_6$-Alkinylthio, $C_2$–$C_6$-Alkinylsulfinyl und $C_2$–$C_6$-Alkinylsulfonyl steht,

$R^1$ für gegebenenfalls durch Halogen, wie Fluor, Chlor oder Brom, $C_1$–$C_2$-Alkyl, $C_1$–$C_2$-Alkoxy, $C_1$–$C_2$-Alkylthio, Halogen-$C_1$–$C_2$-Alkyl, Halogen-$C_1$–$C_2$-Alkoxy, Halogen-$C_1$–$C_2$-Alkylthio, Cyano und/oder Nitro substituiertes Aryl mit 6 bis 10 Kohlenstoffatomen oder für Alkyl mit 1 bis 6 Kohlenstoffatomen steht und

$R^2$ für Wasserstoff oder für gegebenenfalls durch Halogen, Cyano, $C_1$–$C_4$-Alkoxy, $C_1$–$C_4$-Alkoxycarbonyl und/oder gegebenenfalls durch Halogen, $C_1$–$C_2$-Alkyl und $C_1$–$C_2$-Alkoxy substituiertes Phenoxy substituiertes Alkyl, Alkoxy und Alkylthio mit jeweils 1 bis 6 Kohlenstoffatomen; für Alkenyl und Alkinyl mit 2 bis 6 Kohlenstoffatomen; für Cycloalkyl mit 2 bis 8 Kohlenstoffatomen; für gegebenenfalls durch Halogen, $C_1$–$C_2$-Alkoxy substituiertes Aryl und Aryloxy mit 6 bis 10 Kohlenstoffatomen steht,

das dadurch gekennzeichnet ist, dass man Isoharnstoff-Derivate der Formel (II)

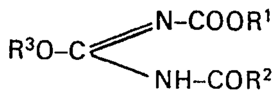

(II)

in welcher

$R^1$ und $R^2$ die oben angegebenen Bedeutungen haben und

$R^3$ für Alkyl steht.

mit Aminen der Formel (III)

$R$–$NH_2$ (III)

in welcher

R die oben angegebenen Bedeutungen hat, gegebenenfalls in Gegenwart einer Säure und gegebenenfalls in Gegenwart eines Verdünnungsmittels umsetzt.

2. Neue Isoharnstoff-Derivate der Formel (IIa).

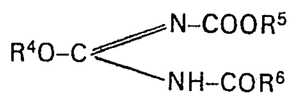

(IIa)

in welcher

$R^4$ für Alkyl mit 1 bis 6 Kohlenstoffatomen steht,

$R^5$ für gegebenenfalls durch Halogen, wie Fluor, Chlor oder Brom, $C_1$–$C_2$-Alkyl, $C_1$–$C_2$-Alkoxy, $C_1$–$C_2$-Alkylthio, Halogen-$C_1$–$C_2$-Alkyl, Halogen-$C_1$–$C_2$-Alkoxy, Halogen-$C_1$–$C_2$-Alkylthio, Cyano und/oder Nitro substituiertes Aryl mit 6 bis 10 Kohlenstoffatomen insbesondere Phenyl oder für Alkyl mit 1 bis 6 Kohlenstoffatomen steht und

$R^6$ für Wasserstoff oder für gegebenenfalls durch Halogen, Cyano, $C_1$–$C_4$-Alkoxy, $C_1$–$C_4$-Alkoxycarbonyl und/oder gegebenenfalls durch Halogen, $C_1$–$C_2$-Alkyl und $C_1$–$C_2$-Alkoxy substituiertes Phenoxy substituiertes Alkyl, Alkoxy und Alkylthio mit jeweils 1 bis 6 Kohlenstoffatomen; für Alkenyl und Alkinyl mit 2 bis 6 Kohlenstoffatomen; für Cycloalkyl mit 3 bis 8 Kohlenstoffatomen; für gegebenenfalls durch Halogen, $C_1$–$C_2$-Alkyl und/oder $C_1$–$C_2$-Alkoxy substituiertes Aryl und Aryloxy mit 6 bis 10 Kohlenstoffatomen steht, wobei jedoch die folgende Verbindung ausgenommen wird: 1,3-Diethoxycarbonyl-2-ethyl-isoharnstoff.

3. Ein neues Verfahren zur Herstellung der Verbindungen der Formel (II), das dadurch gekennzeichnet ist, dass man

(a) Isoharnstoffe der Formel (IV)

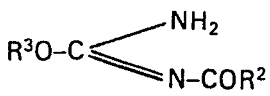

(IV)

in welcher

$R^2$ und $R^3$ die oben (unter 1.) angegebenen Bedeutungen haben,

oder ihre Säureadditionssalze mit Chlorameisensäureestern der Formel (V)

$Cl\text{-}COOR^1$ (V)

in welcher

$R^1$ die oben (unter 1.) angegebenen Bedeutungen hat,

gegebenenfalls in Gegenwart von Säureakzeptoren und gegebenenfalls in Gegenwart von Verdünnungsmitteln umsetzt, oder

(b) Isoharnstoffe der Formel (VI)

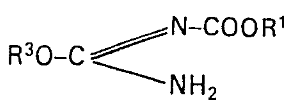

(VI)

in welcher

$R^1$ und $R^3$ die oben (unter 1.) angegebenen Bedeutung haben,

oder ihre Säureadditionssalze, mit Halogeniden der Formel (VII)

$R^2\text{–}CO\text{–}Hal$ (VII)

in welcher

$R^2$ die oben (unter 1.) angegebenen Bedeutungen hat und

Hal für Halogen steht,

gegebenenfalls in Gegenwart von Säureakzeptoren und gegebenenfalls in Gegenwart von Verdünnungsmitteln umsetzt.

Überraschenderweise können mit Hilfe des erfindungsgemässen Verfahrens (1) substituierte Guanidine der Formel (I) ohne eine aufwendige Reinigung und somit kostengünstiger hergestellt werden als aus dem Stand der Technik bekannt ist. Nach dem Stand der Technik werden als Ausgangsprodukte für die Herstellung der Verbindungen der Formel (I) Isothioharnstoffe eingesetzt. Beim Einsatz von Isothioharnstoffen entstehen jedoch Alkylmercaptane als Nebenprodukte. Diese verursachen durch ihre Geruchsintensität eine starke Umweltbelastung und machen eine aufwendige Reinigung der Endprodukte erforderlich (vgl. z.B. EP-OS 27 646 und DE-AS 2 423 679).

Mit Hilfe des erfindungsgemässen Verfahrens (1) werden bevorzugt diejenigen Verbindungen der Formel (I) hergestellt, in welcher

R für einen Rest 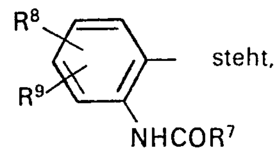 steht, wobei

$R^7$ für Wasserstoff, für gegebenenfalls durch Fluor, Chlor, Brom, Cyano, $C_1$–$C_4$-Alkoxy, $C_1$–$C_4$-Alkoxycarbonyl und/oder gegebenenfalls durch Fluor, Chlor, Brom, $C_1$–$C_2$-Alkyl und/oder $C_1$–$C_2$-Alkoxy substituiertes Phenyl oder Phenoxy substituiertes $C_1$–$C_4$-Alkyl; für $C_2$–$C_4$-Alkenyl; $C_2$–$C_4$-Alkinyl; sowie für gegebenenfalls durch Fluor, Chlor, Brom, $C_1$–$C_2$-Alkyl und/oder $C_1$–$C_2$-Alkoxy substituiertes Phenyl und Phenoxy steht,

$R^8$ für Wasserstoff, Fluor, Chlor, Brom, Methyl, Ethyl, Methoxy oder Ethoxy steht und

$R^9$ für Wasserstoff oder für gegebenenfalls durch Fluor, Chlor, Brom, Cyano, Nitro, Amino, Methyl, Ethyl, Methoxy, Ethoxy, Methylthio, Ethylthio, Trifluormethyl, Trifluormethoxy, Trifluormethylthio; Methylcarbonylamino und/oder Ethylcarbonylamino substituierte Reste aus der Reihe Phenyloxy, Phenylthio, Benzoyl, Phenylsulfinyl und Phenylsulfonyl; sowie für gegebenenfalls durch Fluor, Chlor, Brom, Cyano, Methoxy, Ethoxy, Methylthio und/oder Ethylthio substituierte Reste aus der Reihe $C_1$–$C_4$-Alkyl, $C_1$–$C_4$-Alkoxy, $C_1$–$C_4$-Alkylthio, $C_1$–$C_4$-Alkylsulfinyl, $C_1$–$C_4$-Alkylsulfonyl, $C_2$–$C_4$-Alkenyloxy, $C_2$–$C_4$-Alkenylthio, $C_2$–$C_4$-Alkenylsulfinyl, $C_2$–$C_4$-Alkenylsulfonyl, $C_2$–$C_4$-Alkinyloxy, $C_2$–$C_4$-Alkinylthio, $C_2$–$C_4$-Alkinylsulfinyl und $C_2$–$C_4$-Alkinylsulfonyl steht,

$R^1$ für gegebenenfalls durch Fluor, Chlor, Brom, Methyl, Ethyl, Methoxy, Ethoxy, Methylthio, Ethylthio, Trifluormethyl, Trifluormethoxy, Trifluormethylthio, Cyano und/oder Nitro substituiertes Phenyl oder für Alkyl mit 1 bis 4 Kohlenstoffatomen steht und

$R^2$ für Wasserstoff oder für gegebenenfalls durch Fluor, Chlor, Brom, Cyano, Methoxy, Ethoxy, Methoxycarbonyl, Ethoxycarbonyl und/oder gegebenenfalls durch Fluor, Chlor, Brom, Methyl, Ethyl, Methoxy und/oder Ethoxy substituiertes Phenoxy substituiertes Alkyl, Alkoxy und Alkylthio mit jeweils 1 bis 4 Kohlenstoffatomen: für Allyl, Propargyl, Cyclohexyl sowie für gegebenenfalls durch Fluor, Chlor, Brom, Methyl, Ethyl, Methoxy und/oder Ethoxy substituiertes Phenyl oder Phenoxy steht.

Verwendet man beispielsweise für das erfindungsgemässe Verfahren (1) als Ausgangsstoffe 2-Amino -5-phenylthio -(2-ethoxy)acetanilid und 1,3-Dimethoxycarbonyl -2-methyl-isoharnstoff, so kann die Reaktion durch das folgende Formelschema wiedergegeben werden:

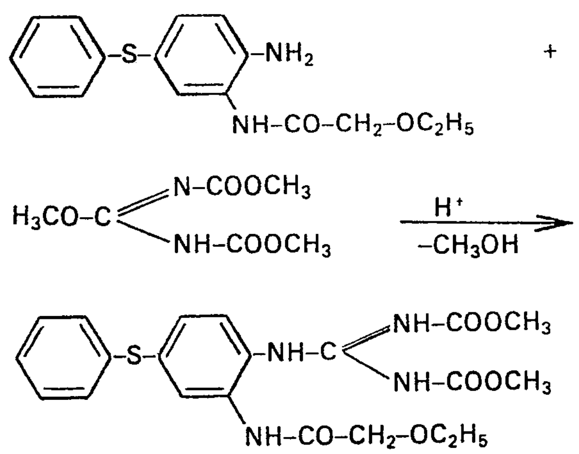


Die als Ausgangsstoffe für das erfindungsgemässe Verfahren (1) zu verwendenden Isoharnstoff-Derivate sind durch die Formel (II) allgemein definiert. In dieser Formel stehen $R^1$, $R^2$ und $R^3$ bevorzugt für diejenigen Reste, welche oben bei der Formel (I) als bevorzugt angegeben sind.

Die Verbindungen der Formel (II) sind bis auf 1,5-Diethoxycarbonyl-2-ethyl-isoharnstoff neu (vgl. z.B. J. Am. Chem. Soc. 48, 2176 (1926) ).

Bevorzugt sind die neuen Verbindungen der Formel (IIa), in welcher

$R^4$ für Alkyl mit 1 bis 4 Kohlenstoffatomen steht,

$R^5$ für gegebenenfalls durch Fluor, Chlor, Brom, Methyl, Ethyl, Methoxy, Ethoxy, Methylthio, Ethylthio, Trifluormethyl, Trifluormethoxy, Trifluormethylthio, Cyano und/oder Nitro substituiertes Phenyl oder für Alkyl mit 1 bis 4 Kohlenstoffatomen steht und

$R^6$ für Wasserstoff oder für gegebenenfalls durch Fluor, Chlor, Brom, Cyano, Methoxy, Ethoxy, Methoxycarbonyl, Ethoxycarbonyl und/oder gegebenenfalls durch Fluor, Chlor, Brom, Methyl, Ethyl, Methoxy und/oder Ethoxy substituiertes Phenoxy substituiertes Alkyl, Alkoxy und Alkylthio mit jeweils 1 bis 4 Kohlenstoffatomen; für Allyl, Propargyl, Cyclohexyl sowie für gegebenenfalls durch Fluor, Chlor, Brom, Methyl, Ethyl, Methoxy und/oder Ethoxy substituiertes Phenyl oder Phenoxy steht, wobei jedoch die folgende Verbindung ausgenommen wird: 1,3-Diethoxycarbonyl-2-ethyl-isoharnstoff.

Die neuen Verbindungen der Formel (IIa) lassen sich gemäss 3 (oben) herstellen (siehe weiter unten).

Die für das erfindungsgemässe Verfahren (1) ausserdem als Ausgangsstoffe zu verwendenden Amine sind durch die Formel (III) allgemein definiert. In dieser Formel steht R bevorzugt für diejenigen Reste, welche oben bei der Formel (I) als bevorzugt angegeben sind.

Die Verbindungen der Formel (III) sind bekannt (vgl. z.B. EP-OS 8438, DE-OS 2 117 293, DE-OS 2 250 911, DE-OS 2 304 764, DE-OS 2 423 679, DE-OS 2 609 994, DE-OS 2 630 847, DE-OS 2 651 467 und DE-OS 2 653 766.

Das erfindungsgemässe Verfahren zur Herstellung der Verbindungen der Formel (I) wird bevorzugt in Gegenwart von polaren Verdünnungsmitteln durchgeführt. Hierzu gehören vorzugsweise Alkohole, wie Methanol, Ethanol, Isopropanol, Ketone, wie Aceton, aber auch Ether wie Dioxan oder Tetrahydrofuran. Gegebenenfalls kann auch in Mischungen derselben mit Wasser gearbeitet werden.

Die bei der Durchführung der Umsetzung von Verbindungen der Formel (II) mit Stoffen der Formel (III) gegebenenfalls als reaktionsfördernde Katalysatoren zugesetzten Säuren können im Prinzip aus der Reihe der bekannten organischen oder anorganischen Säuren beliebig ausgewählt werden. Vorteilhaft verwendet man jedoch die leicht zugänglichen, technisch bedeutenden Vertreter dieser Klassen. Als Beispiel seien genannt: Salzsäure, Schwefelsäure, Salpetersäure, Ameisensäure, Essigsäure, p-Toluolsulfonsäure.

Die Reaktionstemperaturen können bei der Durchführung des erfindungsgemässen Verfahrens (1) in einem grösseren Bereich variiert werden. Im allgemeinen arbeitet man zwischen 0 °C und 120 °C, vorzugsweise zwischen 10° und 30 °C. Die Umsetzung wird im allgemeinen bie Normaldruck durchgeführt.

Bei der Durchführung des erfindungsgemässen Verfahrens (1) werden die Ausgangsstoffe der Formel (II) und (III) gewöhnlich annähernd in äquimolaren Mengen eingesetzt. Ein Überschuss des einen oder anderen Reaktionspartners bringt keine wesentlichen Vorteile. Die Isolierung der Endprodukte erfolgt in allgemein üblicher Weise. Die Verbindungen der Formel (I) fallen beim Abkühlen des Reaktionsgemisches meist kristallin an und können durch Absaugen abgetrennt und gegebenenfalls durch Umlösen bzw. Umkristallisieren gereinigt werden.

Verwendet man beispielsweise für das erfindungsgemässe Verfahren (3), gemäss Variante (a) 2-Methyl -1-phenylcarbonyl-isoharnstoff und Chlorameisensäuremethylester bzw. gemäss Variante (b) 1-Methoxycarbonyl-2-methyl-isoharnstoff und Benzoylchlorid als Ausgangsstoffe, so können die Reaktionen durch die folgenden Formelschemata wiedergegeben werden:

(a)

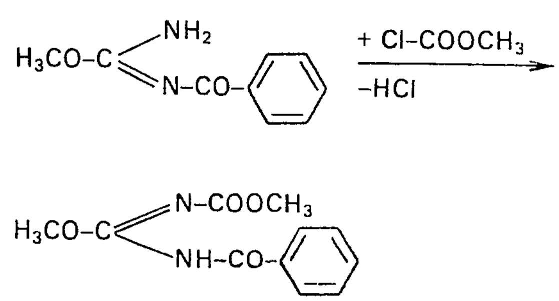

(b)

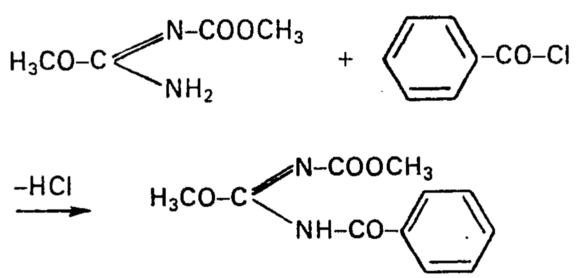

Die als Ausgangsstoffe für das erfindungsgemässe Verfahren (3, Variante a und b) zu verwendenden Isoharnstoffe sind durch die Formel (IV) bzw. (VI) allgemein definiert. In diesen Formeln stehen $R^2$ bzw. $R^1$ und $R^3$ bevorzugt für diejenigen Reste, welche oben bei der Formel (I) als bevorzugt angegeben sind.

Als Ausgangsstoffe für das erfindungsgemässe Verfahren (3, Variante a und b) können auch die Säureadditionssalze aus Säuren und denjenigen Isoharnstoffen der Formel (IV) bzw. (VI), in denen $R^2$ bzw. $R^1$ und $R^3$ die Bedeutungen haben, die bereits vorzugsweise für diese Substituenten genannt wurden, eingesetzt werden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure und 1,5-Naphthalindisulfonsäure.

Die Verbindungen der Formel (IV) bzw. (VI) sind bekannt (vgl. z.B. Synthesis 1979, S. 561 ff, EP-OS 8438 und DE-OS 2 245 449).

Die weiterhin als Ausgangsstoffe für das erfindungsgemässe Verfahren (3, Variante a) zu verwendenden Chlorameisensäureester sind durch die Formel (V) allgemein definiert. In dieser Formel steht $R^1$ bevorzugt für diejenigen Reste, welche oben bei der Formel (I) als bevorzugt angegeben sind.

Die ausserdem als Ausgangsstoffe für das erfindungsgemässe Verfahren (3, Variante b) zu verwendenden Halogenide sind durch die Formel (VII) allgemein definiert. In dieser Formel steht $R^2$ bevorzugt für diejenigen Reste, welche oben bei der Formel (I) als bevorzugt angegeben sind. Hal steht in dieser Formel für Halogen, wie insbesondere für Fluor, Chlor und Brom.

Die Verbindungen der Formel (V) bzw. (VII) sind bekannte Verbindungen der organischen Chemie.

Überraschenderweise können mit Hilfe des erfindungsgemässen Verfahrens (3) Isoharnstoff-Derivate der Formel (II) auf einfache und preisgünstige Weise, in guten Ausbeuten und sehr hoher Reinheit hergestellt werden. So ist bereits bekannt, dass man 1,5-Diethoxycarbonyl-2-ethylisoharnstoff erhält, wenn man das teure Silbersalz von 1,3-Diethoxycarbonyl-isoharnstoff mit Ethyliodid 28 Stunden unter Rückfluss erhitzt (vgl. J. Am. Chem. Soc. 48, 2176 (1926) ). Vorteilhaft ist das erfindungsgemässe Verfahren ausserdem, weil es auch im technischen Massstab anwendbar ist und man auf teure Zwischenprodukte verzichten kann.

Das erfindungsgemässe Verfahren (3. Variante a und b) wird vorzugsweise in Gegenwart von inerten Verdünnungsmitteln durchgeführt.

Hierzu gehören insbesondere aliphatische und aromatische, gegebenenfalls halogenierte Kohlenwasserstoffe, wie Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methylethyl -, Methylisopropyl- und Methylisobutylketon, Ester, wie Essigsäuremethylester und -ethylester. Das erfindungsgemässe Verfahren kann jedoch auch ohne Verdünnungsmittel durchgeführt werden.

Das erfindungsgemässe Verfahren (3) wird in beiden Varianten (a) und (b) vorzugsweise in Gegenwart von Säureakzeptoren durchgeführt werden. Als Säureakzeptoren können alle üblichen Säurebindemittel Verwendung finden. Besonders bewährt haben sind Alkalihydroxide, wie Natrium- und Kaliumhydroxid, ferner aliphatische, aromatische oder heterocyclische Amine, beispielsweise Triethylamin, Trimethylamin, Dimethylanilin, Dimethylbenzylamin und Pyridin.

Das erfindungsgemässe Verfahren (3) wird im allgemeinen bei Temperaturen zwischen −30 °C und +40 °C durchgeführt. Bevorzugt wird der Bereich zwischen −20 °C und +30 °C. Die Umsetzungen werden im allgemeinen bei Normaldruck durchgeführt.

Zur Durchführung des erfindungsgemässen Verfahrens (3) werden in beiden Varianten (a) und (b) die Ausgangsstoffe gewöhnlich in äquimolaren Mengen eingesetzt. Die Umsetzung wird im allgemeinen in einem geeigneten Verdünnungsmittel oder auch ohne Verdünnungsmittel in Gegenwart eines Säureakzeptors durchgeführt und das Reaktionsgemisch wird bei der erforderlichen Temperatur gerührt. Danach gibt man z.B. Wasser zu und arbeitet die organische Phase wie üblich durch Waschen, Trocknen und Abdestillieren des Lösungsmittels auf.

Die Verbindungen der Formel (I) und deren Salze mit anorganischen Säuren weisen eine sehr gute anthemintische Wirkung auf (vgl. EP-OS 8438, DE-OS 2 250 911. DE-OS 2 304 764, DE-OS 2 423 679, DE-OS 2 609 994, DE-OS 2 630 847, DE-OS 2 651 467 und DE-OS 2 653 766). Teilweise können die Verbindungen der Formel (I) auch als Herbizide eingesetzt werden (vgl. z.B. DE-OS 3 328 502).

Herstellungsbeispiele

Beispiel 1

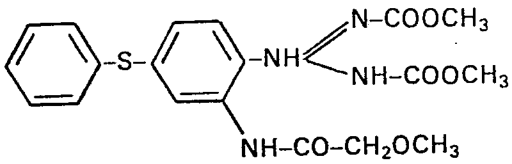

Eine Lösung aus 5,8 g (0,02 Mol) 2-Amino-5-phenylthio-(2-methoxy)-acetanilid, 4,2 g (0,022 Mol) 1,1-Dimethoxycarbonyl-2-methyl-isoharnstoff, 50 ml Methanol und 3 ml Eisessig wird 20 Stunden bei 20 °C gerührt. Das ausgefallene farblose, kristalline Reaktionsprodukt wird abgesaugt und getrocknet.

Man erhält so 6,8 g (76% der Theorie) 1,2-Dimethoxycarbonyl-3-[2-(2-methoxy)-acetamido-5-phenylthio]-guanidin einer 98,5%igen Reinheit und vom Schmelzpunkt 129 °C.

Ausgangsprodukte der Formel (IIa)

Beispiel (IIa-1)

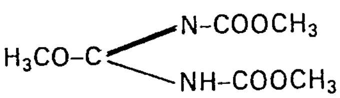

(Verfahren 3a, 1. Variante)

Zu einer Lösung aus 26,4 g (0,2 Mol) 1-Methoxycarbonyl-2-methyl-isoharnstoff, 17,4 g (0,22 Mol) Pyridin werden langsam bei −20 °C bis −10 °C 20,8 g (0,22 Mol) Chlorameisensäuremethylester in 50 ml Chloroform getropft. Das Reaktionsgemisch wird bei −10 °C bis −5 °C noch 1 Stunde nachgerührt, mit Wasser versetzt und die organische Phase abgetrennt. Anschliessend wird das Lösungsmittel destillativ entfernt.

Man erhält so 33,8 g (85% der Theorie) 1,1-Dimethoxycarbonyl-2-methyl-isoharnstoff von einer 96%igen Reinheit und vom Schmelzpunkt 58–59 °C.

(Verfahren 3a, 2. Variante)

Einer Lösung aus 66 g (0,5 Mol) 1-Methoxycarbonyl-2-methyl-isoharnstoff, 350 ml Aceton werden bei 0 °C 73 g (1,1 Mol) festes Kaliumhydroxid, 85%ig, hinzugesetzt. Bei dieser Temperatur werden in das Reaktionsgemisch unter gutem Rühren 100 g (1,06 Mol) Chlorameisensäuremethylester getropft. Der Ansatz wird über Nacht bei 0–10 °C nachgerührt, das Salz abgesaugt und die Lösung eingeengt. Es werden 91 g (96% d. Theorie) 1,1-Dimethoxy-carbonyl-2-methyl-isoharnstoff vom Schmelzpunkt 56–58 °C erhalten.

Beispiel (IIa-2)

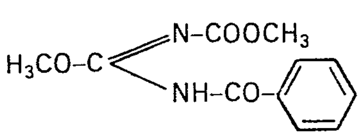

(Verfahren 3b)

Zu einer Lösung aus 13,2 g (0,1 Mol) 1-Methoxycarbonyl-2-methyl-isoharnstoff, 8 g (0,1 Mol) Pyridin und 60 ml Chloroform werden langsam bei −15 °C 14 g (0,1 Mol) Benzoylchlorid getropft. Das Reaktionsgemisch wird ca. 16 Stunden nachgerührt, wobei die Temperatur auf +20 °C ansteigt. Anschliessend wird mit Wasser versetzt, die organische Phase abgetrennt und das Lösungsmittel abdestilliert.

Nach Umkristallisation aus Petrolether erhält man 13 g (58% der Theorie) 1-Methoxycarbonyl-2-methyl-3-phenylcarbonyl-isoharnstoff als farbloses kristallines Pulver vom Schmelzpunkt 101 °C.

Analog Beispiel (IIa-1) und (IIa-2) bzw. Verfahrensvariante 3a und 3b können die folgenden Verbindungen der Formel (IIa) erhalten werden:

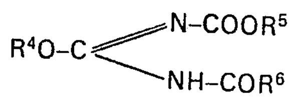

(IIa)

Beispiel (IIa-3)

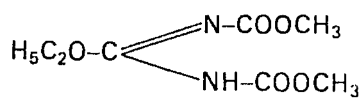

Fp.: 81–82 °C

Beispiel (IIa-4)

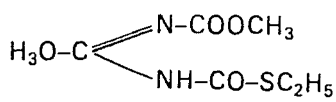

Ausbeute: 21% der Theorie; Fp.: 42–43 °C

Beispiel (IIa-5)

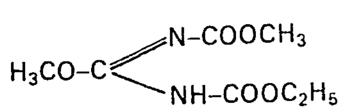

Ausbeute: 52,3% der Theorie; Fp.: 54–55 °C

Gemäss dem erfindungsgemässen Verfahren 3 (Variante a und b) lässt sich auch die bekannte Verbindung der Formel (II): 1,3-Diethoxycarbonyl-2-ethyl-isoharnstoff (J. Am. Chem. Soc. 48, 2176 (1926) ) auf einfache und kostengünstige Weise und in guten Ausbeuten (80% der Theorie) herstellen.

## Patentansprüche

1. Verfahren zur Herstellung von substituierten Guanidinen der Formel (I).

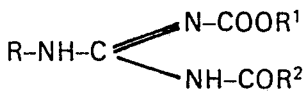

(I)

in welcher

R für einen Rest 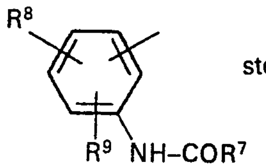 steht, wobei

$R^7$ für Wasserstoff oder für gegebenenfalls substituierte Reste aus der Reihe $C_{1-6}$-Alkyl, $C_{2-7}$-Alkenyl, $C_2$–$C_6$-Alkinyl, $C_1$–$C_6$-Alkoxy, $C_3$–$C_8$-Cycloalkyl, Phenyl und Phenoxy steht,

$R^8$ für Wasserstoff, Halogen, $C_1$–$C_4$-Alkyl oder $C_1$–$C_4$-Alkoxy steht und

$R^9$ für Wasserstoff oder für gegebenenfalls durch Halogen, Cyano, Nitro, Amino, $C_1$–$C_4$-Alkyl, $C_1$–$C_4$-Alkoxy, $C_1$–$C_4$-Alkylthio, Halogen-$C_1$–$C_4$-Alkyl, Halogen-$C_1$–$C_4$-Alkoxy, Halogen-$C_1$–$C_4$-Alkylthio und/oder $C_1$–$C_4$-Alkylcarbonylamino substituierte Reste aus der Reihe Phenoxy, Phenylthio, Benzoyl, Phenylsulfinyl und Phenylsulfonyl; sowie für gegebenenfalls durch Halogen, Cyano, $C_1$–$C_4$-Alkoxy und/oder $C_1$–$C_4$-Alkylthio substituierte Reste aus der Reihe $C_1$–$C_6$-Alkyl, $C_1$–$C_6$-Alkoxy, $C_1$–$C_6$-Alkylthio, $C_1$–$C_6$-Alkylsulfinyl, $C_1$–$C_6$-Alkylsulfonyl, $C_2$–$C_6$-Alkenyloxy, $C_2$–$C_6$-Alkenylthio, $C_2$–$C_6$-Alkenylsulfinyl, $C_2$–$C_6$-Alkenylsulfonyl, $C_2$–$C_6$-Alkinyloxy, $C_2$–$C_6$-Alkinylthio, $C_2$–$C_6$-Alkinylsulfinyl und $C_2$–$C_6$-Alkinylsulfonyl steht,

$R^1$ für gegebenenfalls durch Halogen, wie Fluor, Chlor oder Brom, $C_1$–$C_2$-Alkyl, $C_1$–$C_2$-Alkoxy, $C_1$–$C_2$-Alkylthio, Halogen-$C_1$–$C_2$-Alkyl, Halogen-$C_1$–$C_2$-Alkoxy, Halogen-$C_1$–$C_2$-Alkylthio, Cyano und/oder Nitro substituiertes Aryl mit 6 bis 10 Kohlenstoffatomen oder für Alkyl mit 1 bis 6 Kohlenstoffatomen steht und

$R^2$ für Wasserstoff oder für gegebenenfalls durch Halogen, Cyano, $C_1$–$C_4$-Alkoxy, $C_1$–$C_4$-Alkoxycarbonyl und/oder gegebenenfalls durch Halogen, $C_1$–$C_2$-Alkyl und $C_1$–$C_2$-Alkoxy substituiertes Phenoxy substituiertes Alkyl, Alkoxy und Alkylthio mit jeweils 1 bis 6 Kohlenstoffatomen; für Alkenyl und Alkinyl mit 2 bis 6 Kohlenstoffatomen; für Cycloalkyl mit 2 bis 8 Kohlenstoffatomen; für gegebenenfalls durch Halogen, $C_1$–$C_2$-Alkoxy substituiertes Aryl und Aryloxy mit 6 bis 10 Kohlenstoffatomen steht, dadurch gekennzeichnet, dass man Isoharnstoff-Derivate der Formel (II)

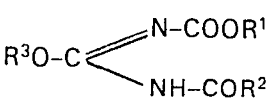

(II)

in welcher

$R^1$ und $R^2$ die oben angegebenen Bedeutungen haben und

$R^3$ für Alkyl steht, mit Aminen der Formel (III)

$R{-}NH_2$ (III)

in welcher

R die oben angegebenen Bedeutungen hat, gegebenenfalls in Gegenwart einer Säure und gegebenenfalls in Gegenwart eines Verdünnungsmittels umsetzt.

2. Isoharnstoff-Derivate der Formel (IIa),

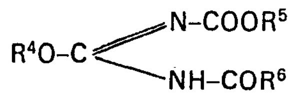

(IIa)

in welcher

$R^4$ für Alkyl mit 1 bis 6 Kohlenstoffatomen steht,

$R^5$ für gegebenenfalls durch Halogen, wie Fluor, Chlor oder Brom, $C_1$–$C_2$-Alkyl, $C_1$–$C_2$-Alkoxy, $C_1$–$C_2$-Alkylthio, Halogen-$C_1$–$C_2$-Alkyl, Halogen-$C_1$–$C_2$-Alkoxy, Halogen-$C_1$–$C_2$-Alkylthio, Cyano und/oder Nitro substituiertes Aryl mit 6 bis 10 Kohlenstoffatomen insbesondere Phenyl oder für Alkyl mit 1 bis 6 Kohlenstoffatomen steht und

$R^6$ für Wasserstoff oder für gegebenenfalls durch Halogen, Cyano, $C_1$–$C_4$-Alkoxy, $C_1$–$C_4$-Alkoxycarbonyl und/oder gegebenenfalls durch Halogen, $C_1$–$C_2$-Alkyl und $C_1$–$C_2$-Alkoxy substituiertes Phenoxy substituiertes Alkyl, Alkoxy und Alkylthio mit jeweils 1 bis 6 Kohlenstoffatomen; für Alkenyl und Alkinyl mit 2 bis 6 Kohlenstoffatomen; für Cycloalkyl mit 3 bis 8 Kohlenstoffatomen; für gegebenenfalls durch Halogen, $C_1$–$C_2$-Alkyl und/oder $C_1$–$C_2$-Alkoxy substituiertes Aryl und Aryloxy mit 6 bis 10 Kohlenstoffatomen steht, wobei jedoch die folgende Verbindung ausgenommen wird; 1,3-Diethoxycarbonyl-2-ethyl-isoharnstoff.

3. Verfahren zur Herstellung der Verbindungen der Formel (II) gemäss Anspruch 2, dadurch gekennzeichnet, dass man

(a) Isoharnstoffe der Formel (IV)

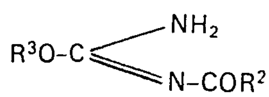

(IV)

in welcher

$R^2$ und $R^3$ die in Anspruch 1 angegebenen Bedeutungen haben, oder ihre Säureadditionssalze, mit Chlorameisensäureestern der Formel (V)

$Cl{-}COOR^1$ (V)

in welcher

$R^1$ die in Anspruch 1 angegebenen Bedeutungen hat, gegebenenfalls in Gegenwart von Säureakzeptoren und gegebenenfalls in Gegenwart von Verdünnungsmitteln umsetzt, oder

(b) Isoharnstoffe der Formel (VI)

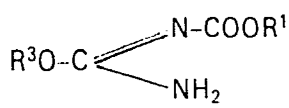

(VI)

in welcher

$R^1$ und $R^3$ die in Anspruch 1 angegebenen Bedeutung haben, oder ihre Säureadditionssalze, mit Halogeniden der Formel (VII)

$R^2$–CO–Hal (VII)

in welcher

$R^2$ die in Anspruch 1 angegebenen Bedeutungen hat und

Hal für Halogen steht, gegebenenfalls in Gegenwart von Säureakzeptoren und gegebenenfalls in Gegenwart von Verdünnungsmitteln umsetzt.

**Revendications**

1. Procédé pour préparer des guanidines substituées de formule (I)

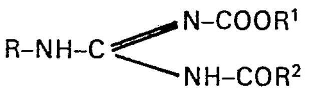

(I)

dans laquelle

R représente un reste 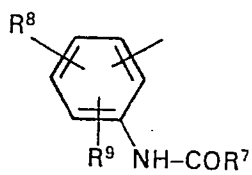

$R^7$ représente un atome d'hydrogène ou des restes, éventuellement substitués, choisis dans la série formée d'un groupe alkyle en $C_1$–$C_6$, alcényle en $C_2$–$C_7$, alcynyle en $C_2$–$C_6$, alcoxy en $C_1$–$C_6$, cycloalkyle en $C_3$–$C_8$, phényle et phénoxy,

$R^8$ représente un atome d'hydrogène ou d'halogène, un groupe alkyle en $C_1$–$C_4$ ou alcoxy en $C_1$–$C_4$, et

$R^9$ représente un atome d'hydrogène ou des restes (éventuellement substitués par de l'halogène, par un groupe cyano, nitro, amino, alkyle en $C_1$–$C_4$, alcoxy en $C_1$–$C_4$, alkylthio en $C_1$–$C_4$, halogéno-alkyle en $C_1$–$C_4$, halogéno-alcoxy en $C_1$–$C_4$, halogénoalkylthio en $C_1$–$C_4$ et/ou alkylcarbonylamino en $C_1$–$C_4$), choisis dans la série comprenant un groupe phénoxy, phénylthio, benzoyle, phénylsulfonyle et phénylsulfonyle; ainsi que des restes (éventuellement substitués par de l'halogène, par un groupe cyano, alcoxy en $C_1$–$C_4$ et/ou alkylthio en $C_1$–$C_4$) choisis parmi la série comprenant un groupe alkyle en $C_1$–$C_6$, alcoxy en $C_1$–$C_6$, alkylthio en $C_1$–$C_6$, alkylsulfinyle en $C_1$–$C_6$, alkylsulfinyle en $C_1$–$C_6$, alcényloxy en $C_2$–$C_6$, alcénylthio en $C_2$–$C_6$, alcénylsulfinyle en $C_2$–$C_6$, alcénylsulfonyle en $C_2$–$C_6$, alcynyloxy en $C_2$–$C_6$, alcynylthio en $C_2$–$C_6$, alcynylsulfinyle en $C_2$–$C_6$ et alcynylsulfonyle,

$R^1$ représente un reste aryle comportant 6 à 10 atomes de carbone (éventuellement substitué par de l'halogène, comme du fluor, du chlore, ou de brome, par un groupe alkyle en $C_1$–$C_2$, alcoxy en $C_1$–$C_2$, alkylthio en $C_1$–$C_2$, halogéno-alkyle en $C_1$–$C_2$, halogéno-alcoxy en $C_1$–$C_2$, halogénoalkylthio en $C_1$–$C_2$, cyano et/ou nitro) ou un reste alkyle ayant 1 à 6 atomes de carbone, et

$R^2$ représente un atome d'hydrogène ou un reste alkyle, alcoxy et alkylthio, ayant chacun 1 à 6 atomes de carbone (et qui sont éventuellement substitués par de l'halogène, un groupe cyano, alcoxy en $C_1$–$C_4$, alcoxycarbonyle en $C_1$–$C_4$ et/ou phénoxy, lui-même éventuellement substitué par de l'halogène, par un groupe alkyle en $C_1$–$C_2$ et alcoxy en $C_1$–$C_2$); un reste alcényle et alcynyle ayant 2 à 6 atomes de carbone; un reste cycloalkyle ayant 2 à 8 atomes de carbone; un reste aryle et aryloxy, comportant 6 à 10 atomes de carbone, (éventuellement substitués par de l'halogène ou par un groupe alcoxy ayant 1 ou 2 atomes de carbone), procédé caractérisé en ce qu'on fait réagir, éventuellement en présence d'un acide et éventuellement en présence d'un diluant, des dérivés de l'isourée, répondant à la formule (II)

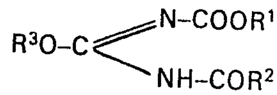

(II)

dans laquelle

$R^1$ et $R^2$ ont les sens indiqués ci-dessus, et

$R^3$ représente un groupe alkyle, avec des amines de formule (III)

$R$–$NH_2$ (III)

dans laquelle

R a les sens indiqués ci-dessus.

2. De nouveaux dérivés de l'isourée, répondant à la formule (IIa).

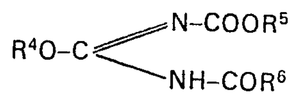

(IIa)

dans laquelle

$R^4$ représente un groupe alkyle ayant 1 à 6 atomes de carbone,

$R^5$ représente un reste aryle ayant 6 à 10 atomes de carbone, en particulier phényle (éventuellement substitués par un atome d'halogène comme le fluor, le chlore ou le brome, par un groupe alkyle en $C_1$–$C_2$, alcoxy en $C_1$–$C_2$, alkylthio en $C_1$–$C_2$, halogénoalkyle en $C_1$–$C_2$, halogénoalcoxy en $C_1$–$C_2$, halogénoalkylthio en $C_1$–$C_2$, cyano et/ou nitro) ou un reste alkyle ayant 1 à 6 atomes de carbone, et

$R^6$ représente un atome d'hydrogène ou un reste alkyle, alcoxy et alkylthio, ayant chacun 1 à 6 atomes de carbone (éventuellement substitués par de l'halogène, par un groupe cyano, alcoxy en $C_1$–$C_4$, alcoxycarbonyle en $C_1$–$C_4$ et/ou phénoxy, lui-même éventuellement substitué par de l'halogène, par un groupe alkyle en $C_1$–$C_2$ et alcoxy en $C_1$–$C_2$); un reste alcényle et alcynyle ayant 2 à 6 atomes de carbone; un reste cycloalkyle ayant 3 à 8 atomes de carbone; un reste aryle et un reste aryloxy, ayant 6 à 10 atomes de carbone (éventuellement substitués par de l'halogène, par un groupe alkyle en $C_1$–$C_2$ et/ou alcoxy en $C_1$–$C_2$), à l'exclusion cependant du composé suivant: la 1,3-diéthoxycarbonyl-2-éthyl-isourée.

3. Procédé pour préparer les composés de formule (II) selon la revendication 2, caractérisé en ce que

(a) on fait réagir, éventuellement en présence d'accepteurs d'acides et éventuellement en présence de diluants, des isourées de formule (IV)

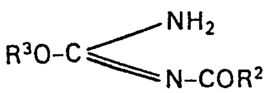
(IV)

dans laquelle

$R^2$ et $R^3$ ont les sens indiqués à la revendication 1, ou leurs sels d'addition d'acide, avec des esters de l'acide chloroformique, répondant à la formule (V)

$Cl-COOR^1$ (V)

dans laquelle

$R^1$ a les sens indiqués à la revendication 1, ou

(b) on fait réagir, éventuellement en présence d'accepteurs d'acides et éventuellement en présence de diluants, des isourées de formule (VI)

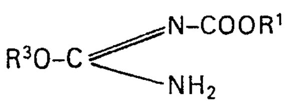
(VI)

dans laquelle

$R^1$ et $R^3$ ont les sens indiqué à la revendication 1, ou leurs sels d'addition d'acide, avec des halogénures de formule (VII)

$R^2-CO-Hal$ (VII)

dans laquelle

$R^2$ a les sens indiqués à la revendication 1, et Hal représente un halogène.

**Claims**

1. Process for the preparation of substituted guanidines of the formula (I)

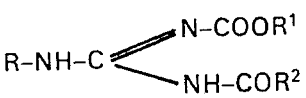
(I)

in which

R represents a radical 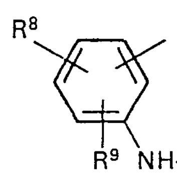

wherein

$R^7$ represents hydrogen or optionally substituted radicals from the series comprising $C_{1-6}$-alkyl, $C_{2-7}$-alkenyl, $C_2$–$C_6$-alkinyl, $C_1$–$C_6$-alkoxy, $C_3$–$C_8$-cycloalkyl, phenyl and phenoxy,

$R^8$ represents hydrogen, halogen, $C_1$–$C_4$-alkyl or $C_1$–$C_4$-alkoxy and

$R^9$ represents hydrogen or radicals from the series comprising phenoxy, phenylthio, benzoyl, phenylsulphinyl and phenylsulphonyl, which are optionally substituted by halogen, cyano, nitro, amino, $C_1$–$C_4$-alkyl, $C_1$–$C_4$-alkoxy, $C_1$–$C_4$-alkylthio, halogeno-$C_1$–$C_4$-alkyl, halogeno-$C_1$–$C_4$-alkoxy, halogeno-$C_1$–$C_4$-alkylthio and/or $C_1$–$C_4$-alkylcarbonylamino; or represents radicals from the series comprising $C_1$–$C_6$-alkyl, $C_1$–$C_6$-alkoxy, $C_1$–$C_6$-alkylthio, $C_1$–$C_6$-alkylsulphinyl, $C_1$–$C_6$-alkylsulphonyl, $C_2$–$C_6$-alkenyloxy, $C_2$–$C_6$-alkenylthio, $C_2$–$C_6$-alkenylsulphinyl, $C_2$–$C_6$-alkenylsulphonyl, $C_2$–$C_6$-alkinyloxy, $C_2$–$C_6$-alkinylthio, $C_2$–$C_6$-alkinylsulphinyl and $C_2$–$C_6$-alkinylsulphonyl, which are optionally substituted by halogen, cyano, $C_1$–$C_4$-alkoxy and/or $C_1$–$C_4$-alkylthio,

$R^1$ represents aryl which has 6 to 10 carbon atoms and is optionally substituted by halogen, such as fluorine, chlorine or bromine, $C_1$–$C_2$-alkyl, $C_1$–$C_2$-alkoxy, $C_1$–$C_2$-alkylthio, halogeno-$C_1$–$C_2$-alkyl, halogeno-$C_1$–$C_2$-alkoxy, halogeno-$C_1$–$C_2$-alkylthio, cyano and/or nitro, or represents alkyl having 1 to 6 carbon atoms, and

$R^2$ represents hydrogen or alkyl, alkoxy and alkylthio, each of which has 1 to 6 carbon atoms and is optionally substituted by halogen, cyano, $C_1$–$C_4$-alkoxy, $C_1$–$C_4$-alkoxycarbonyl and/or by phenoxy which is optionally substituted by halogen, $C_1$–$C_2$-alkyl and $C_1$–$C_2$-alkoxy; or represents alkenyl and alkinyl having 2 to 6 carbon atoms; cycloalkyl having 2 to 8 carbon atoms; or optionally halogen-substituted or optionally $C_1$–$C_2$-alkoxy-substituted aryl and aryloxy having 6 to 10 carbon atoms, characterised in that isourea derivatives of the formula (II)

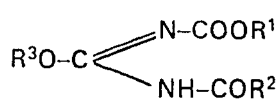
(II)

in which

$R^1$ and $R^2$ have the meanings given above and

$R^3$ represents alkyl, are reacted with amines of the formule (III)

$R-NH_2$ (III)

in which

R has the meanings given above, if appropriate in the presence of an acid and, if appropriate, in the presence of a diluent.

2. Isourea derivatives of the formula (IIa)

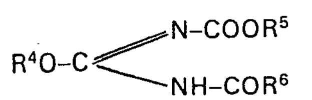
(IIa)

in which

$R^4$ represents alkyl having 1 to 6 carbon atoms,

$R^5$ represents aryl which is optionally substituted by halogen, such as fluorine, chlorine or bromine, $C_1$–$C_2$-alkyl, $C_1$–$C_2$-alkoxy, $C_1$–$C_2$-alkylthio, halogeno-$C_1$–$C_2$-alkyl, halogeno-$C_1$–$C_2$-alkoxy, halogeno-$C_1$–$C_2$-alkylthio, cyano and/or nitro and has 6 to 10 carbon atoms, in particular phenyl, or represents alkyl having 1 to 6 carbon atoms and

$R^6$ represents hydrogen or alkyl, alkoxy and alkylthio, each of which has 1 to 6 carbon atoms and is optionally substituted by halogen, cyano, $C_1$–$C_4$-alkoxy, $C_1$–$C_4$-alkoxycarbonyl and/or by phenoxy which is optionally substituted by halogen, $C_1$–$C_2$-alkyl and $C_1$–$C_2$-alkoxy; or represents alkenyl and alkinyl having 2 to 6 carbon atoms; cycloalkyl having 3 to 8 carbon atoms; or optionally halogen-substituted, $C_1$–$C_2$-alkyl-substituted and/or $C_1$–$C_2$-alkoxy-substituted aryl and aryloxy having 6 to 10 carbon atoms,
with the exception, however, of the following compound: 1,3-diethoxycarbonyl- 2-ethyl-isourea.

3. Process for the preparation of the compounds of the formula (II) according to Claim 2, characterised in that

(a) isoureas of the formula (IV)

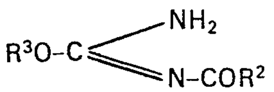

(IV)

in which

$R^2$ and $R^3$ have the meanings given in Claim 1, or their acid addition salts, are reacted with chloroformates of the formula (V)

$Cl–COOR^1$ (V)

in which

$R^1$ has the meanings given in Claim 1, if appropriate in the presence of acid acceptors and, if appropriate, in the presence of diluents, or

(b) isoureas of the formula (VI)

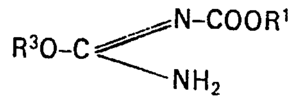

(VI)

in which

$R^1$ and $R^3$ have the meanings given in Claim 1, or their acid addition salts, are reacted with halides of the formula (VII)

$R^2–CO–Hal$ (VII)

in which

$R^2$ has the meanings given in Claim 1 and Hal represents halogen,

if appropriate in the presence of acid acceptors and, if appropriate, in the presence of diluents.